# FASCICULE DE BREVET EUROPEEN

(11) **EP 2 353 902 B1**
(45) Date de publication et mention de la délivrance du brevet: **22.08.2012**
(21) Numéro de dépôt: 11305036.3
(22) Date de dépôt: 14.01.2011
(51) Int. Cl.: B60H 3/00

(54) **Système de désinfection ou de désodorisation et véhicule automobile équipé d'un tel système**
Desinfektions- oder Desodorisierungssystem, und mit einem solchen System ausgestattetes Kraftfahrzeug
System for disinfecting or deodorising an automobile provided with such a system

(30) Priorité: 14.01.2010 FR 1050231
(43) Date de publication de la demande: 10.08.2011
(73) Titulaire: Clim Assistance, 69210 Fleurieux sur L'arbresle (FR)
(72) Inventeur: Planques, Guillaume, 69290 POLLIONNAY (FR); Lagarrigue, Thierry, 69490 SARCEY (FR)
(74) Mandataire: Palix, Stéphane

(56) Documents cités:
- WO-A1-2005/075895
- DE-U- 1 811 309
- DE-U- 1 842 221
- DE-U1- 20 321 495

## Description

### DOMAINE TECHNIQUE

La présente invention se rapporte au domaine de la désinfection ou de la désodorisation de véhicules automobiles. De tels véhicules peuvent notamment se présenter sous la forme de voitures, de vans ou d'autobus étant équipés d'un système de production d'énergie pneumatique, de façon à alimenter en air comprimé certains organes du véhicule, tel que des portes ou le siège du conducteur de façon à améliorer le confort lors de la conduite du véhicule.

L'invention vise plus particulièrement des systèmes de désinfection ou de désodorisation permettant de traiter le véhicule quotidiennement, notamment le soir après son utilisation.

### ART ANTERIEUR

De façon générale, les systèmes de désinfection ou de désodorisation destinés à équiper des véhicules automobiles, comprennent un réservoir d'air comprimé de façon à utiliser l'air comprimé pour acheminer une substance désodorisante ou désinfectante à l'intérieur de canalisations.

Par suite, de tels systèmes nécessitent le remplacement régulier de la cartouche d'air comprimé une fois violée.

Selon un autre mode de réalisation, les systèmes de désinfection peuvent également comporter une source d'énergie pneumatique autonome intégrée pour générer de l'air comprimé.

DE 1811309 U décrit un système selon le préambule de la revendication 1.

Par conséquent, ce type de système de désinfection présente un volume d'encombrement important qui ne permet pas de l'adapter à tous les véhicules automobiles. En effet, les véhicules automobiles présentant un volume de chargement de taille réduite ne peuvent être équipés de tels systèmes de désinfection ou de désodorisation.

Ainsi, le but de l'invention est de réaliser un système de désinfection ou de désodorisation nécessitant peu d'opérations de maintenance et étant particulièrement compact et pouvant être agencé à l'intérieur de n'importe quel véhicule automobile présentant un système de production d'énergie pneumatique.

### EXPOSE DE L'INVENTION

L'invention concerne donc un système de désinfection ou de désodorisation destiné à être agencé dans un véhicule automobile équipé d'un système de production d'énergie pneumatique. Un tel système de désinfection ou de désodorisation comporte un réservoir apte à contenir une substance désinfectante ou désodorisante.

Ce système de désinfection ou de désodorisation se **caractérise en ce qu**'il comporte :
- des moyens de connexion permettant de mettre en communication le système de production d'énergie pneumatique du véhicule avec tout ou partie du réservoir ;
- au moins une canalisation pour acheminer la substance désinfectante ou désodorisante du réservoir jusqu'à une buse de pulvérisation de la substance, cette substance étant déplacée dans la canalisation par de l'air comprimé généré par le système de production d'énergie pneumatique du véhicule ;
- au moins un compartiment de dosage et une électrovanne de dosage pour remplir ce compartiment de dosage avec la substance désinfectante ou désodorisante contenue dans le réservoir.

Autrement dit, les moyens de connexion sont solidarisés avec le système de production d'énergie pneumatique et permettent d'utiliser l'air comprimé généré par le véhicule qui peut être notamment stocké dans une bouteille dite *« de servitude* ».

Cet air comprimé permet alors de déplacer la substance désinfectante à l'intérieur d'une canalisation et une buse de pulvérisation permet de disperser la substance dans le véhicule.

Par ailleurs, l'utilisation du compartiment de dosage permet de n'utiliser qu'une partie de la substance désinfectante ou désodorisante contenue dans le réservoir, et de calibrer la quantité exacte utilisée à chaque cycle de désinfection. Le remplissage du compartiment de dosage peut notamment s'effectuer en dehors du cycle proprement dit de désinfection qui peut être réalisé ultérieurement. Par exemple, ce remplissage du compartiment de dosage peut être réalisé lors d'une mise en route du véhicule, tandis que le cycle de désinfection proprement dit s'effectuera une fois le véhicule arrêté, par exemple pendant la nuit.

Avantageusement, le système de désinfection ou de désodorisation peut comporter une électrovanne de commande pour commander la pulvérisation de la substance par la buse au moyen de l'air comprimé généré par le système de production d'énergie pneumatique du véhicule.

En d'autres termes, lorsque l'électrovanne de commande est au repos, la pulvérisation de la substance n'est pas effectuée. Par ailleurs, dans une position activée de l'électrovanne de commande, celle-ci permet de générer la circulation de l'air comprimé à l'intérieur de la canalisation et d'acheminer la substance désinfectante jusqu'à la buse de pulvérisation.

Une telle électrovanne de commande peut en outre être pilotée manuellement par un interrupteur actionné par le conducteur du véhicule ou automatiquement par une unité de contôle permettant de temporiser le déclenchement de la désinfection/désodorisation du véhicule.

Par ailleurs, le système de désinfection ou de désodorisation peut comporter une électrovanne de mise à l'air libre du compartiment de dosage pour permettre le remplissage du compartiment de dosage avec la substance désinfectante ou désodorisante et l'évacuation de la pression générée par l'air comprimé présent dans la canalisation.

De cette manière, l'air contenu dans le compartiment de dosage peut être évacué et la substance désinfectante ou désodorisante peut alors remplir le compartiment de dosage par la simple action de la force gravitationnelle. De même, une fois le cycle de désinfection réalisé, cette électrovanne de mise à l'air libre permet d'évacuer l'air comprimé contenu dans les canalisations et ainsi de porter à la pression atmosphérique l'air résiduel dans les canalisations.

Selon un mode de réalisation particulier, le système de désinfection peut comporter un dispositif de régulation de la pression de l'air comprimé généré par le système de production d'énergie pneumatique du véhicule.

Un tel dispositif de régulation permet notamment de réaliser une détente de l'air comprimé contenu dans la bouteille de servitude, mais également de générer une pulvérisation de la substance à pression constante par les buses de pulvérisation.

Selon un premier mode de réalisation, la buse de pulvérisation de la substance peut être agencée dans l'habitacle du véhicule.

Dans ce cas, la substance de désinfection est alors pulvérisée à l'intérieur du volume interne du véhicule et permet de traiter l'air contenu à l'intérieur de ce volume. Le système de ventilation du véhicule est alors également activé de façon à brasser l'air de l'habitacle et désinfecter l'air contenu dans les conduites de ventilation.

Selon un second mode de réalisation, la buse de pulvérisation de la substance peut être agencée à proximité d'un organe de climatisation de l'air de ventilation du véhicule. En effet, dans ce cas, la buse permet de traiter un élément tel que l'évaporateur au travers duquel l'air de ventilation circule et est ensuite acheminé à l'intérieur du compartiment du véhicule par des conduites de ventilation. Ainsi, l'air contenu dans l'habitacle de véhicule est traité ou désinfecté dans un deuxième temps au moyen de l'air chargé en substance désinfectante ayant circulé à l'intérieur des conduites de ventilation.

Par ailleurs, il est également envisageable de combiner plusieurs buses et notamment de les agencer à différentes positions situées soit dans l'habitacle soit à proximité d'un organe de climatisation de l'air de ventilation du véhicule.

Avantageusement, le système de désinfection ou de désodorisation peut comporter un organe de commande agencé dans un compartiment de pilotage du véhicule et permettant de commander la mise en service d'un cycle de désinfection ou de désodorisation du véhicule.

Autrement dit, le conducteur du véhicule peut actionner un organe de commande lorsqu'il souhaite activer un cycle de désinfection ou de désodorisation du véhicule. Cette commande peut également être conditionnée par l'arrêt du véhicule et par conséquent, la coupure du contact au moyen de la clé de démarrage du véhicule. Ainsi, une fois le contact coupé et l'organe de commande actionné, un cycle de désinfection peut être lancé par le conducteur qui sort de son véhicule une fois sa journée de travail effectuée ou son trajet quotidien terminé.

Par ailleurs, la commande des différentes électrovannes du système de désinfection peut être réalisée de différentes manières et notamment par une technologie électrique dans laquelle des relais de temporisation peuvent être utilisés ou encore une technologie électronique permettant une grande flexibilité.

Ainsi, le système de désinfection ou de désodorisation peut comporter une unité de contrôle électronique permettant de commander au moins une électrovanne. Une telle unité de contrôle électronique permet alors d'effectuer une temporisation et une synchronisation de l'activation des différentes électrovannes. Elle permet également de piloter le démarrage d'un cycle de désinfection par des moyens automatiques, tels que des éléments de commande à distance utilisant des ondes de type infrarouge, ou toute autre technologie de communication sans fil tel que le WIFI^{®} ou le BLUE TOOTH^{®}.

L'unité de contrôle électronique permet alors de piloter indépendamment chaque électrovanne en fonction des données d'entrée générées par le conducteur du véhicule ou par une source externe, tel qu'une borne WIFI^{®} agencée au niveau d'une zone de parking du véhicule.

Comme déjà évoqué, l'invention concerne également un véhicule automobile équipé d'un système de production d'énergie pneumatique permettant d'alimenter en air comprimé une bouteille dite "de servitude" et comportant un système de désinfection ou de désodorisation tel que précédemment décrit. Un tel système de désinfection ou de désodorisation est par ailleurs connecté à la bouteille de servitude par les moyens de connexion.

Ainsi, un véhicule automobile est équipé de moyens de connexion permettant de mettre en communication la bouteille « de servitude » avec un réservoir ou un compartiment de dosage dans lequel la substance désinfectante est agencée.

Un tel véhicule automobile comporte également au moins une canalisation pouvant acheminer la substance désinfectante depuis le réservoir jusqu'à une buse de pulvérisation de la substance.

### DESCRIPTION SOMMAIRE DES FIGURES

La manière de réaliser l'invention ainsi que les avantages qui en découlent, ressortiront bien du mode de réalisation qui suit, donné à titre indicatif mais non limitatif, à l'appui des figures annexées dans lesquelles :
- la figure 1 représente une vue en perspective d'un véhicule équipé du système de désinfection ou de désodorisation conforme à l'invention ;
- les figures 2 à 6 représentent schématiquement le système de désinfection ou de désodorisation et les différents états de ses composants pneumatiques lors de son fonctionnement ;
- la figure 7 représente une vue en perspective d'un agencement particulier des buses de pulvérisation.

### MANIERE DE DECRIRE L'INVENTION

Comme déjà évoqué, l'invention concerne un système de désinfection ou de désodorisation destiné à être agencé dans un véhicule automobile.

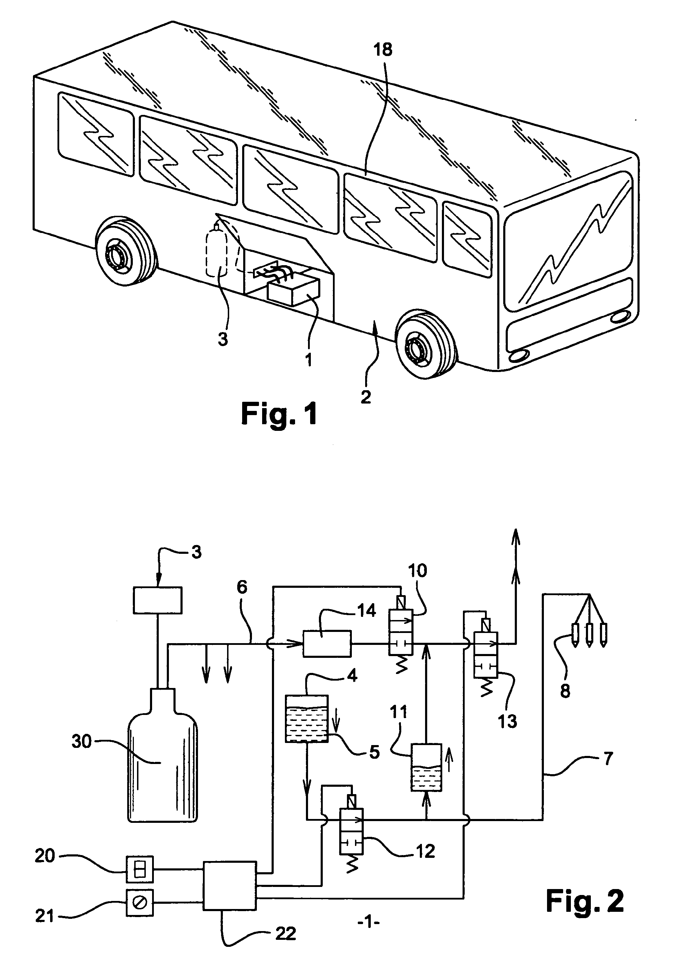

Tel que représenté à la figure 1, un tel système de désinfection ou de désodorisation **1** peut être placé dans un compartiment indépendant, tel que la soute d'un véhicule automobile **2,** ici représenté sous la forme d'un autocar. Par ailleurs, ce type de véhicule automobile **2** est équipé d'un système de production d'énergie pneumatique **3** permettant d'alimenter des accessoires du véhicule, tel que l'ouverture des portes notamment. Un tel système de désinfection permet ainsi de traiter l'habitacle **18** du véhicule lorsque celui-ci n'est pas utilisé, par exemple la nuit, dans son parking.

Tel que représenté à la figure 2, un tel système de désinfection 1 est connecté à un système de production d'énergie pneumatique **3** du véhicule, pour utiliser de l'air comprimé étant constamment disponible grâce à une source d'énergie autonome et équipant le véhicule. Un tel véhicule peut également comporter une bouteille dite de servitude **30** dans laquelle la pression en air comprimé est régulée par le système de production d'énergie pneumatique **3.**

Des moyens de connexion **6** permettent alors d'utiliser cet air comprimé stocké dans la bouteille de servitude **30,** puis l'air comprimé passe dans un dispositif de régulation **14** permettant d'abaisser la pression de l'air comprimé à une valeur notamment comprise entre 6 et 8 bars.

Ce système de désinfection **1** comporte également un réservoir **4** permettant de contenir une substance désinfectante désodorisante **5.** La substance **5** circule ensuite dans au moins une canalisation **7** jusqu'à au moins une buse de pulvérisation **8** qui peut être placée dans l'habitacle du véhicule.

Par ailleurs, le système de désinfection **1** comporte également une unité de contrôle électronique **22** permettant d'actionner une électrovanne de commande **10** permettant d'injecter l'air comprimé dans le circuit, une électrovanne de dosage **12** permettant de remplir un compartiment de dosage **11** avec la substance désinfectante, et une électrovanne de mise à l'air libre **13.**

De plus, le dispositif peut comporter un organe de commande **20** agencé dans le compartiment de pilotage du véhicule **2** et un organe de sécurité **21** formé par la clé de démarrage du véhicule ou par un relai, dont l'état est fonction de la position de la clé de démarrage du véhicule.

Ainsi, lorsque le contact est coupé au niveau de l'organe de sécurité **21,** et que l'utilisateur presse l'organe de commande **20,** l'unité de contrôle électronique **22** peut commencer le cycle de désinfection du véhicule.

Tel que représenté à la figure 2, lors de la mise en route du véhicule au moyen de l'organe de sécurité **21,** l'unité de contrôle électronique **22** peut notamment commander le remplissage du réservoir de dosage **11** avec la substance désinfectante contenue dans le réservoir **4.** Pour cela, elle commande l'ouverture de l'électrovanne de dosage **12** et de l'électrovanne de mise à l'air libre **13.** Ainsi, la substance désinfectante **5** peut s'écouler par gravité dans la canalisation et remplir le compartiment de dosage **11.** L'air contenu dans le compartiment de dosage **11** est alors libre de s'échapper par un évent en traversant préalablement l'électrovanne de mise à l'air libre **13.** Ce remplissage du compartiment de dosage **11** peut donc s'effectuer lors de la circulation du véhicule.

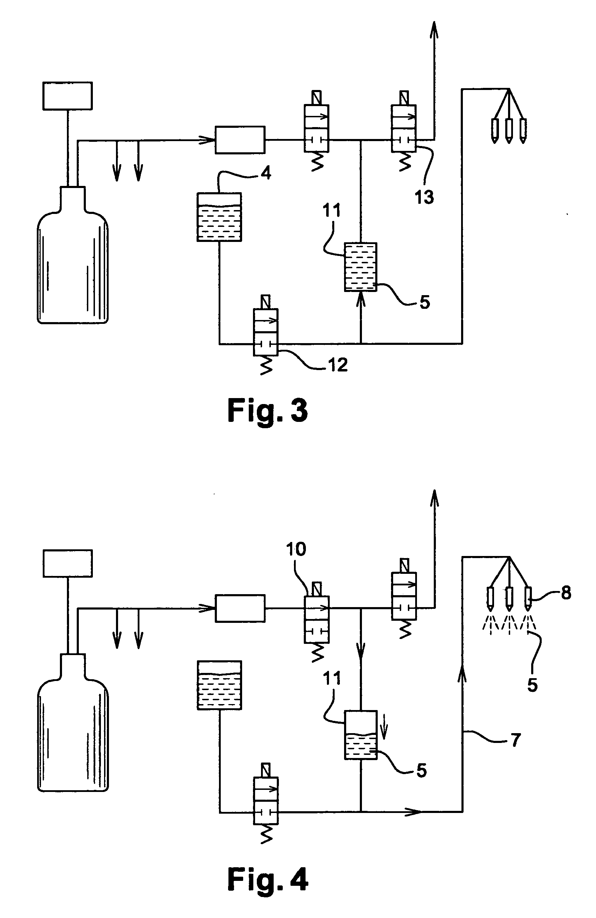

Tel que représenté à la figure 3, une fois le réservoir de dosage **11** rempli, l'unité de commande **22** ferme l'électrovanne de dosage **12** et l'électrovanne de mise à l'air libre **13.**

Ensuite et tel que représenté à la figure 4, la pulvérisation peut être déclenchée en ouvrant l'électrovanne de commande **10,** permettant d'injecter l'air comprimé dans le circuit en passant au travers du réservoir du compartiment de dosage **11,** puis dans la canalisation **7** pour acheminer la substance jusqu'aux buses de pulvérisation **5.**

Cet état d'activation de l'électrovanne de commande **10** peut notamment durer près de huit minutes en perdurant, une fois la substance pulvérisée, afin de supprimer toute gouttelette de substance désinfectante dans les canalisations **7.** Tel que représenté, le système de pulvérisation peut comporter trois buses **8** reliées au moyen de trois tuyaux de dérivation de longueur identique et de section identique, présentant chacun par exemple un diamètre intérieur de 2 mm, reliés à une canalisation **7** de diamètre intérieur de 4 mm.

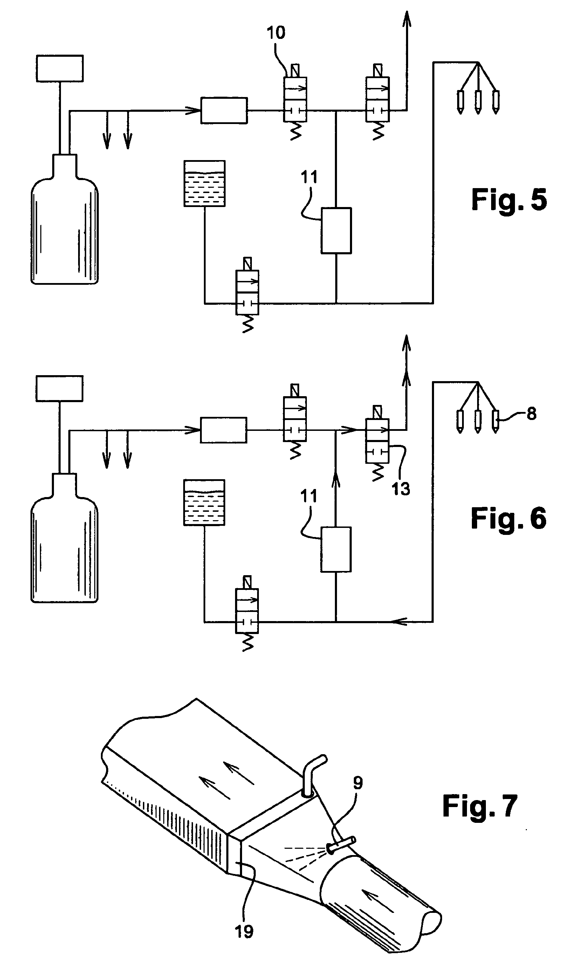

Une fois le cycle de désinfection terminé, l'électrovanne de commande **10** est fermée, puis tel que représenté à la figure 6, l'électrovanne de mise à l'air libre **13** est ouverte afin d'évacuer la pression résiduelle dans les canalisations et de rétablir la pression atmosphérique.

Tel que représenté à la figure 7, une buse de pulvérisation **9** peut également être agencée à proximité d'un organe de climatisation **19** de l'air en ventilation du véhicule. L'air est ensuite acheminé à l'intérieur des conduits de ventilation du véhicule et permet de désinfecter la totalité de l'habitacle du véhicule.

Il ressort de ce qui précède qu'un système de désinfection et un véhicule automobile conformes à l'invention présentent de nombreux avantages, et notamment :
- ils permettent de traiter et de désinfecter tout véhicule équipé d'un système autonome de production d'énergie pneumatique ;
- il nécessite un volume de stockage réduit et peut être facilement inséré dans tout véhicule ;
- il est rapidement connectable et déconnectable avec tout système de production d'énergie pneumatique ;
- il peut être ainsi facilement remplacé lors d'une panne du système notamment.

## Revendications

1. Système de désinfection ou de désodorisation (1) destiné à être agencé dans un véhicule automobile (2) équipé d'un système de production d'énergie pneumatique (3), ledit système de désinfection ou de désodorisation (1) comportant un réservoir (4) apte à contenir une substance désinfectante ou désodorisante (5) comprenant :
• des moyens de connexion (6) permettant de mettre en communication ledit système de production d'énergie pneumatique (3) du véhicule (2) avec tout ou partie du réservoir (4);
• au moins une canalisation (7) pour acheminer la substance désinfectante ou désodorisante (5) du réservoir (4) jusqu'à une buse de pulvérisation (8,9) de la substance (5), ladite substance (5) étant déplacée dans ladite au moins une canalisation (7) par de l'air comprimé généré par le système de production d'énergie pneumatique (3) du véhicule (2) ; **caractérisé en ce qu'**il comporte :
• au moins un compartiment de dosage (11) et une électrovanne de dosage (12) pour remplir ledit compartiment de dosage (11) avec la substance désinfectante ou désodorisante (5) contenue dans le réservoir (4).

2. Système de désinfection ou de désodorisation selon la revendication 1, ***caractérisé* en ce qu'**il comporte une électrovanne de commande (10) pour commander la pulvérisation de la substance (5) par la buse (8) au moyen de l'air comprimé généré par le système de production d'énergie pneumatique (3) du véhicule (2).

3. Système de désinfection ou de désodorisation selon la revendication 1, ***caractérisé* en ce qu'**il comporte une électrovanne de mise à l'air libre (13) du compartiment de dosage (11) pour permettre le remplissage du compartiment de dosage (11) avec la substance désinfectante ou désodorisante (5) et l'évacuation de la pression générée par l'air comprimé présent dans la canalisation (7).

4. Système de désinfection ou de désodorisation selon la revendication 1, ***caractérisé* en ce qu'**il comporte un dispositif de régulation (14) de la pression de l'air comprimé généré par le système de production d'énergie pneumatique (3) du véhicule (2).

5. Système de désinfection ou de désodorisation selon la revendication 1, ***caractérisé* en ce que** ladite buse de pulvérisation (8) de la substance (5) est agencée dans l'habitacle (18) du véhicule (2).

6. Système de désinfection ou de désodorisation selon la revendication 1, ***caractérisé* en ce que** ladite buse de pulvérisation (9) de la substance est agencée à proximité d'un organe de climatisation (19) de l'air de ventilation du véhicule (2).

7. Système de désinfection ou de désodorisation selon la revendication 1, ***caractérisé* en ce qu'**il comporte un organe de commande (20) agencé dans un compartiment de pilotage du véhicule (2) et permettant de commander la mise en service d'un cycle de désinfection ou de désodorisation du véhicule (2).

8. Système de désinfection ou de désodorisation selon l'une des revendications 1 à 3, ***caractérisé* en ce qu'**il comporte une unité de contrôle électronique (22) permettant de commander au moins une électrovanne (10, 12, 13).

9. Véhicule automobile équipé d'un système de production d'énergie pneumatique permettant d'alimenter en air comprimé une bouteille dite "de servitude" et ***caractérisé* en ce qu'**il comporte un système de désinfection ou de désodorisation (1) selon l'une des revendications précédentes, ledit système de désinfection ou de désodorisation (1) étant connecté à ladite bouteille de servitude (30) par les moyens de connexion (6).

## Claims

1. A disinfection or deodorizationsystem (1) intended to be arranged in a motor vehicle (2) equipped with a pneumatic energygenerating system (3), said disinfecting or deodorizing system (1) comprising a tank (4) adapted to contain a disinfecting or deodorizing substance (5)comprising:
- connection means (6) for placing said pneumatic energy generation system (3) of the vehicle (2) in communication with all or part of the tank (4);
- at least one pipe (7) for conveying the disinfecting or deodorizing substance (5) from the tank (4) to a spray nozzle (8,9) for spraying the substance (5), said substance (5) being conveyed in said at least one pipe (7) by compressed air generated by the pneumatic energy generation system (3) of the vehicle (2);
**characterized in that** it comprises:
- at least one dosing chamber (11) and a metering solenoid valve (12) to fill said dosing chamber (11) with the disinfecting or deodorizing substance (5) contained in the reservoir (4).

2. The disinfection or deodorization system according to claim 1, **characterized in that** it comprises a control solenoid valve (10) for controlling the spraying of the substance (5) by the nozzle (8) by means of compressed air generated by the pneumatic energy generation system (3) of the vehicle (2).

3. The disinfection or deodorization system according to claim 1, **characterized in that** it comprises a vent solenoid valve (13) forventing the dosing chamber (11) to allow filling of the dosing chamber (11) with the disinfecting or deodorizing substance (5) and evacuating the pressure generated by the compressed air present in the pipe (7).

4. The disinfection or deodorization system according to claim 1, **characterized in that** it comprises a device (14) for regulating the compressed air pressure generated by the pneumatic energy generation system (3) of the vehicle (2).

5. The disinfection or deodorization system according to claim 1, **characterized in that** said spray nozzle (8) for spraying the substance (5) is arranged in the passenger compartment (18) of the vehicle (2).

6. The disinfection or deodorization system according to claim 1, **characterized in that** said spray nozzle (9) for spraying the substance is arranged close to an air conditioningmember(19) for the ventilation air of the vehicle (2).

7. The disinfection or deodorization system according to claim 1, **characterized in that** it comprises a control member (20) arranged in a vehicle driving compartment (2) for controlling the initiation of a disinfection or deodorizing cycle of the vehicle (2).

8. The disinfection or deodorization system according toany one of claims 1 to 3, **characterized in that** it comprises an electronic control unit (22) for controlling at least one solenoid valve (10, 12, 13).

9. A motor vehicle equipped with a pneumatic energy generation system for supplying compressed air to a socalled "utility" bottle, **characterized in that** it comprises a disinfection or deodorizationsystem (1) according to any one of the preceding claims, wherein said disinfection or deodorizationsystem (1) is connected to said utility bottle (30) by the connecting means (6).

## Patentansprüche

1. Desinfektions- oder Desodorisierungssystem (1), das dazu bestimmt ist, in einem Kraftfahrzeug (2) angeordnet zu sein, das mit einem pneumatischen Energieerzeugungssystem (3) ausgestattet ist, wobei das Desinfektionsoder Desodorisierungssystem (1) einen Behälter (4) umfasst, der dazu ausgelegt ist, eine desinfizierende oder desodorierende Substanz (5) zu enthalten, Folgendes umfassend:
• Verbindungseinrichtungen (6), die es ermöglichen, das pneumatische Energieerzeugungssystem (3) des Fahrzeugs (2) mit dem gesamten oder einem Teil des Behälters (4) in Verbindung zu setzen;
• mindestens eine Rohrleitung (7), um die desinfizierende oder desodorisierende Substanz (5) aus dem Behälter (4) bis zu einer Zerstäubungsdüse (8, 9) für die Substanz (5) zu leiten, wobei die Substanz (5) in der mindestens einen Rohrleitung (7) durch die Druckluft fortbewegt wird, die durch das pneumatische Energieerzeugungssystem (3) des Fahrzeugs (2) erzeugt wird;
**dadurch gekennzeichnet, dass** es umfasst:
• mindestens ein Dosierungsfach (11) und ein Dosierungselektroventil (12), um das Dosierungsfach (11) mit der im Behälter (4) enthaltenen desinfizierenden oder desodorisierenden Substanz (5) zu füllen.

2. Desinfektions- oder Desodorisierungssystem nach Anspruch 1, **dadurch gekennzeichnet, dass** es ein Steuerelektroventil (10) umfasst, um die Zerstäubung der Substanz (5) durch die Düse (8) mittels der durch das pneumatische Energieerzeugungssystem (3) des Fahrzeugs (2) erzeugten Druckluft zu steuern.

3. Desinfektions- oder Desodorisierungssystem nach Anspruch 1, **dadurch gekennzeichnet, dass** es ein Elektroventil (13) zur Entlüftung des Dosierungsfachs (11) umfasst, um die Befüllung des Dosierungsfachs (11) mit der desinfizierenden oder desodorisierenden Substanz (5) und das Ausleiten des durch die in der Rohrleitung (7) vorhandene Druckluft erzeugten Drucks zu ermöglichen.

4. Desinfektions- oder Desodorisierungssystem nach Anspruch 1, **dadurch gekennzeichnet, dass** es eine Regelvorrichtung (14) für den Druck der durch das pneumatische Energieerzeugungssystem (3) des Fahrzeugs (2) erzeugten Druckluft umfasst.

5. Desinfektions- oder Desodorisierungssystem nach Anspruch 1, **dadurch gekennzeichnet, dass** die Zerstäubungsdüse (8) für die Substanz (5) im Fahrgastraum (18) des Fahrzeugs (2) angeordnet ist.

6. Desinfektions- oder Desodorisierungssystem nach Anspruch 1, **dadurch gekennzeichnet, dass** die Zerstäubungsdüse (9) für die Substanz in der Nähe eines Klimatisierungselements (19) für die Belüftungsluft des Fahrzeugs (2) angeordnet ist.

7. Desinfektions- oder Desodorisierungssystem nach Anspruch 1, **dadurch gekennzeichnet, dass** es ein Steuerelement (20) umfasst, das in einem Steuerabteil des Fahrzeugs (2) angeordnet ist und es ermöglicht, die Inbetriebsetzung eines Desinfektions- oder Desodorisierungszyklus des Fahrzeugs (2) zu steuern.

8. Desinfektions- oder Desodorisierungssystem nach einem der Ansprüche 1 bis 3, **dadurch gekennzeichnet, dass** es eine elektronische Steuereinheit (22) umfasst, die es ermöglicht, mindestens ein Elektroventil (10, 12, 13) zu steuern.

9. Kraftfahrzeug, das mit einem pneumatischen Energieerzeugungssystem ausgestattet ist, das es ermöglicht, eine Flasche, "Zusatzflasche" genannt, mit Druckluft zu versorgen, und **dadurch gekennzeichnet ist, dass** es ein Desinfektions- oder Desodorisierungssystem (1) nach einem der vorhergehenden Ansprüche umfasst, wobei das Desinfektions- oder Desodorisierungssystem (1) mittels der Verbindungseinrichtungen (6) an die Zusatzflasche (30) angeschlossen ist.
